# EUROPEAN PATENT SPECIFICATION

(11) **EP 0 544 485 B1**
(45) Date of publication and mention of the grant of the patent: **08.03.1995**
(21) Application number: 92310691.8
(22) Date of filing: 23.11.1992
(51) Int. Cl.: A61B 17/00, A61F 2/00

(54) **Tissue aperture repair device**
Vorrichtung zum Wiederherstellen einer Gewebeöffnung
Appareil de réparation d'une ouverture dans un tissu

(30) Priority: 25.11.1991 US 797321; 25.11.1991 DK 1914/91
(43) Date of publication of application: 02.06.1993
(73) Proprietor: Cook Incorporated, Bloomington Indiana 47402 (US)
(72) Inventor: Gianturco, Cesare, Chamaign, Illinois 61821 (US); Himpens, Jacques, B-9921 Lowendegim (BE); Dilling-Hansen, Jorgen, DK-4000 Roskilde (DK)
(74) Representative: Johnston, Kenneth Graham

(56) References cited:
- EP-A- 0 362 113
- WO-A-92/06639
- DE-A- 2 822 603
- US-A- 3 874 388
- US-A- 5 108 420

## Description

This invention relates to tissue aperture repair apparatus.

To prevent a hernia from recurring, a barrier material is commonly positioned over a hernia ring and affixed to the surrounding tissue to block passage of the peritoneum therethrough and to strengthen or reinforce the abdominal wall about the hernia ring.

Strengthening or reinforcing of hernias is traditionally accomplished by stitching a piece of tissue across a hernial ring. With recent minimally invasive surgical procedures, hernial ring repair is percutaneously performed with the aid of an endoscope, or a laparoscope, and one or more trocar access sheaths inserted into the abdominal cavity, thereby avoiding a much more invasive procedure such as open surgery with accompanying trauma to surrounding tissue.

U.S.-A-3 874 388 discloses a tissue aperture repair device as defined in the preamble of claim 1.

According to the present invention, there is provided apparatus as claimed in claim 1.

The elastic stiffener of the first embodiment may be directly attached to the foldable sheet utilizing attachment means such as suture material, biocompatible adhesive, clips, etc. The stiffener can also be woven through the foldable sheet about the circumference thereof.

The stiffener advantageously expands the foldable sheet and maintains it in its unfolded shape.

The tissue aperture repair device is advantageously used in both open surgery and minimally invasive laparoscopic procedures and further includes affixation means such as a helical fastener insertable through the foldable sheet or suture material extending from the sheet for affixing the foldable sheet of material in its unfolded shape to tissue positioned about the aperture.

The stiffener advantageously comprises an elastic wire such as stainless steel or a superelastic wire of a nickel-titanium alloy such as nitinol for expanding and conforming to the shape of the device cavity and the outer circumference of the foldable sheet of material. The ends of the stiffener are shaped to prevent snagging or catching on the foldable and second sheets of material. The shape of the device cavity and the outer circumference are advantageously elliptical or circular in shape to present a smooth, evenly tensioned device surface for attachment to tissue about the aperture. The stiffener advantageously eliminates the problem of unwrapping and positioning wetted prosthetic mesh in both open surgery and minimally invasive endoscopic procedures.

After introduction into the peritoneal cavity during a minimally invasive procedure, the introducer of the device is detached from the unfolded sheets of material by engaging the collar or the material about the cavity opening against the distal end of the sheath while withdrawing the introducer therefrom. An affixation suture extending from the positioned, unfolded sheets of material is percutaneously drawn through the tissue aperture to fixedly position the device over the tissue aperture for subsequent tissue ingrowth. Affixation means such as helical coil fasteners or suture material are positioned through the unfolded material sheets to affix the repair device to tissue surrounding the hernial ring. The extending suture can be percutaneously affixed to the patient's skin for sole or additional device affixation or removed after positioning of the repair device and affixation to surrounding tissue with other affixation means. The foldable sheet of material positioned against the tissue aperture advantageously provides for the ingrowth of repair tissue.

### Brief Description of the Drawing

FIG. 1 depicts the inguinal canal viewed from the inside of the abdominal cavity;
FIG. 2 is a corresponding illustration after the tissue aperture repair device according to the present invention has been positioned;
FIG. 3 depicts a partially sectioned pictorial view of an illustrative tissue aperture repair device of the present invention;
FIG.4 depicts a partially sectioned side view of the tissue aperture repair device of FIG.3 with a percutaneous introducer inserted therein;
FIG.5 depicts a partially sectioned view of the tissue aperture repair device of FIG.4 in a folded condition and positioned in a containment sheath;
FIG.6 depicts a partially sectioned view of the repair device of FIG.4 in an unfolded shape and a stiffener being inserted therein for maintaining the device in the unfolded shape;
FIG.7 depicts an enlarged view of the wire coil fastener of FIG.6 interconnecting the proximal end of the stiffener and the distal end of the wire guide;
FIG.8 depicts an illustrative helical coil fastener and application cannula for affixing the repair device of FIG.3 to tissue surrounding the tissue aperture or hernial ring;
FIG.9 is a corresponding illustration after the supporting device according to another embodiment has been fitted;

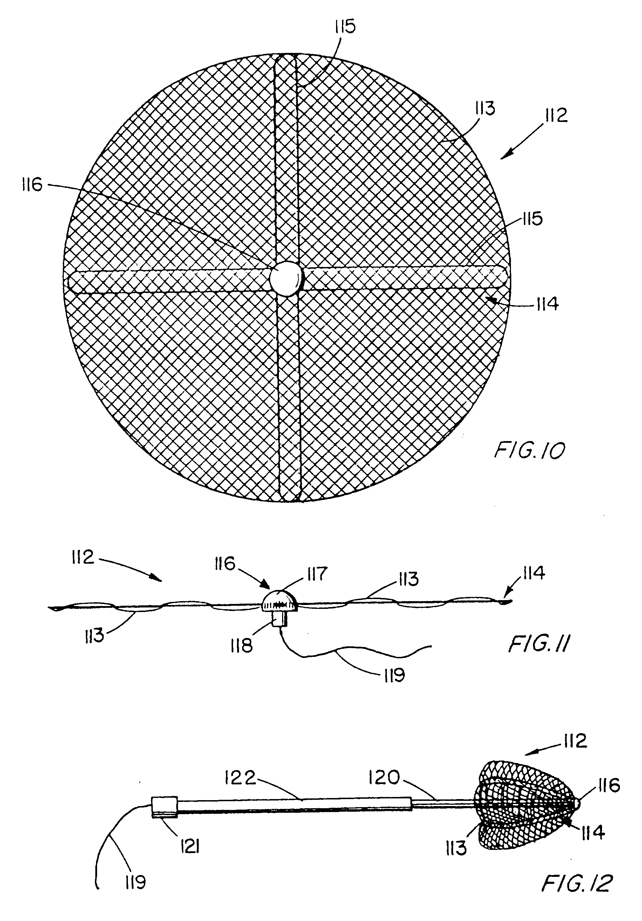

FIGs.10 and 11 are a plane view and a side elevation, respectively, of the supporting device;
FIG.12 is a side elevation of an apparatus with a supporting device mounted thereon; and
FIGs.13 to 16 illustrate how the supporting device may be inserted and positioned during the surgical operation; and
FIGs.17 to 20 illustrate another embodiment of the invention.

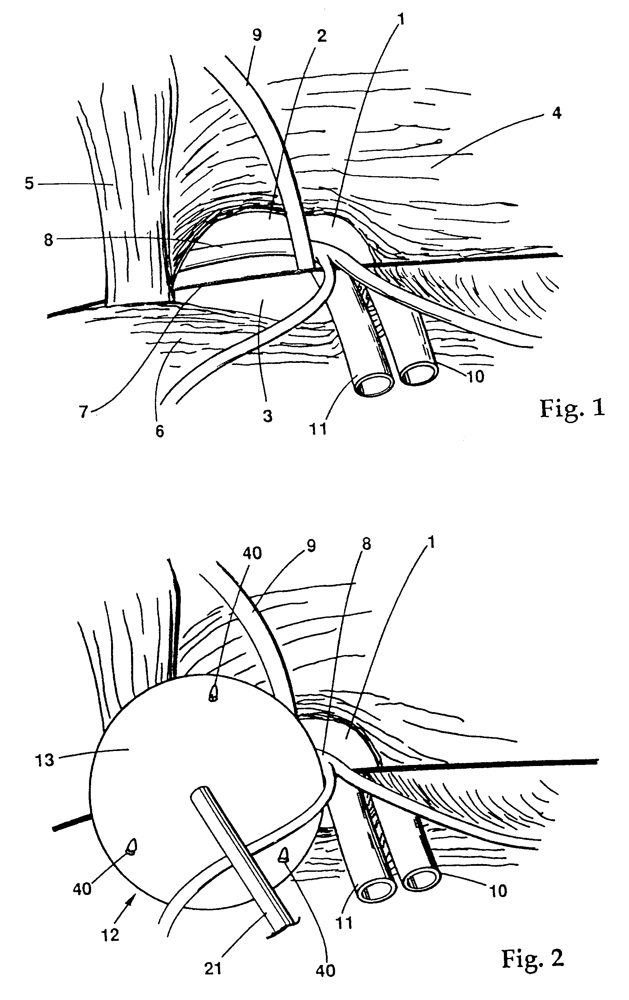

Depicted in FIG. 1 is the inguinal canal in a male patient. The peritoneum and the transversal fascia have not been shown for the sake of clarity. Three potential tissue apertures, particularly hernial rings 1 and 2 for hernia inguinalis and ring 3 for hernia femoralis are indicated. Musculature 4 reflects the location of musculus obliquus internus and musculus transversalis, while musculature 5 reflects the location of musculus rectus. Hernial ring 3 is delimited by pubis and Cooper's ligament 6 and by inguinal ligament 7, which also delimits the two other tissue apertures or hernial rings. Spermatic cord 8 extends upwards through the inguinal canal past epigastric vessel 9 in Hasselbach's ligament and into the abdominal cavity. Femoral artery 10 and vein 11 are also shown.

After a hernia is formed, the musculature around the inguinal canal is weakened, thereby increasing the risk of recurrent hernia. This may be eliminated by inserting, as depicted in FIG. 2, a tissue aperture repair device 12 across the major part of the tissue aperture or hernial ring, thereby preventing the peritoneum from penetrating through one of hernial rings 1-3. Device 12 has an unfolded elliptical or circular shape for abutting on part of the musculature of the abdominal cavity to strengthen it and for at least partially covering tissue apertures or hernial rings 1-3. As a result, spermatic cord 8, femoral artery 10 and vein 11 together with epigastric vessel 9 may concurrently pass freely by the tissue aperture repair device.

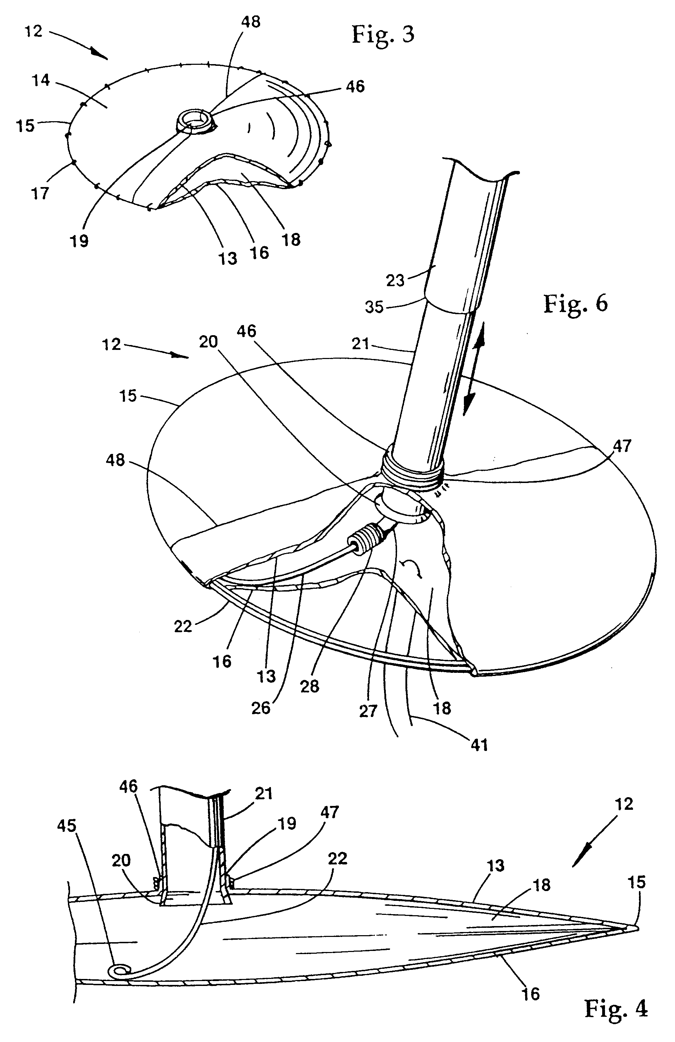

Depicted in FIG. 3 is a partially sectioned pictorial view of illustrative tissue aperture repair device 12 for at least partially covering a tissue aperture such as a hernial ring. The repair device includes foldable sheet 13 of material having unfolded circular shape 14 and circumference 15. When the repair device is used in open surgery, the material of the foldable sheet preferably comprises, for example, a porous prosthetic mesh material such as "MARLEX"® material or other commercially available biocompatible materials for reinforcing or strengthening the tissue positioned about the tissue aperture or hernial ring and for providing a structure for tissue ingrowth about the aperture. "MARLEX"® mesh material is a 6 mil monofilament knitted polypropylene material. The diameter of the unfolded circular shape is approximately 7 cm for at least partially covering the tissue aperture. Diameter sizes can vary preferably in the range from 5 to 10 cm. Preferably, only one tissue aperture repair device should be used to cover the tissue aperture or hernial ring. However, depending on the size and shape of the tissue aperture, several repair devices can be positioned over the aperture and overlapped for providing sufficient coverage of the aperture as desired by the attending physician. The unfolded shape of the sheet can also assume an elliptical shape for at least partially covering elongated tissue apertures or hernial rings. Centrally positioned opening 19 is formed in a single sheet of foldable material for inserting a stiffener therethrough as will be described hereinafter.

When the repair device is used in a minimally invasive laparoscopic procedure, the material of the foldable sheet preferably comprises, for example, a thin, "DACRON"® or nylon, filtering or particulated material, which, for example, is commercially available from Tetco Incorporated, Briar Cliff Manor, New York, as their PE7-53/32 material. This thin, particulated sheet of material is easily folded or rolled tightly about an introducer for containment within a sheath and introduction through a trocar access sheath, which is commonly used in laparoscopic procedures. Foldable sheet 13 is formed from two sheets of the particulated material abutted and heat sealed to form centrally positioned and extending collar 46 with opening 19 therein, as depicted in FIG. 3. Seam 48 in foldable sheet 13 is formed by heat sealing the abutted ends of the two sheets of particulated material.

The tissue aperture repair device also includes support means such as a second sheet 16 of material attached about circumference 15 of foldable sheet 13 for maintaining the foldable sheet in unfolded circular shape 14. The foldable and second sheets 13 and 16 of material are attached about circumference 15 of the foldable sheet using a well-known heat seal process. When the repair device is used in open surgery and the integrity of the circumferential seal is a concern to the surgeon, suture material 17 is stitched about the circumference for added strength and support. Second sheet 16 of material also comprises a sheet of prosthetic mesh such as "MARLEX"® material heat sealed to the foldable sheet of material about the circumference thereof. It is contemplated that a tightly woven or solid nonporous sheet of material such as silicone, GORTEX polymer material, polytetrafluoroethylene, or other commercially available biocompatible material can be used for preventing tissue ingrowth when positioned, for example, against the organs contained within the peritoneal or other internal cavity. This nonporous material may also be biodegradable. However, dog experiments have shown that preventing tissue ingrowth on the surface of the repair device facing internal organs is extremely difficult. A flap of the peritoneum positioned over the affixed repair device is preferred for preventing adhesions to internal organs. Virtual device cavity 18 is formed between foldable and second sheets 13 and 16 of material that are circumferentially attached together. The outer radial shape of device cavity 18 abuts circumference 15 and assumes unfolded circular shape 14 when a stiffener such as an elastic wire is inserted in the cavity.

The tissue aperture repair device also includes a stiffener for applying force to the foldable sheet of material to maintain it in an unfolded shape or condition. The length of the stiffener is selected to be longer than the circumference of the device to conform to unfolded circumference 15 when inserted in device cavity 18. The elastic stiffener wire may comprise a length of stainless steel wire approximately five times the length of the device circumference or a length of a nickel-titanium alloy wire such as commercially available nitinol preferably at least the length of the device circumference. When used as an open surgery repair device, both ends of the stiffener wire are bent into tight closed loops and soldered to prevent snagging. The ends of the wire are also curved to better conform the wire to the circumference of the cavity. When used in a minimally invasive procedure, the proximal end of the stiffener is attached to a wire guide for introduction into the device cavity.

Although device cavity 18 is illustrated as a flat circular disk when in the unfolded state, it is contemplated that the cavity may assume other shapes or configurations for receiving a stiffener for applying force to the foldable sheet of material for maintaining the foldable sheet in the unfolded shape. In particular, device cavity 18 can be formed as a circular ring with a length of narrow width material attached circumferentially about the foldable sheet of material. The internal edge of the narrow width material would be likewise attached about the circumference of the foldable sheet a short distance inward from circumference 15. The stiffener is then inserted into this circular ring device cavity for applying force for maintaining the foldable sheet in the unfolded shape. The stiffener in this instance could also be a straight length of elastic wire at least longer than the circumference of the device. Alternatively, the stiffener could also assume the shape of an elastic wire coil for providing even further flexibility for use in open surgery. It is also contemplated that a plurality of pockets of the second material could be diagonally attached to the foldable sheet and straight stiffeners be inserted therebetween for stretching the foldable sheet to the unfolded circular shape. When the second sheet of material does not completely cover the foldable sheet of material, the exposed side of the foldable sheet of material should inhibit tissue ingrowth. In such case, the foldable sheet can be multi-layered with one layer promoting tissue ingrowth and the other layer of the sheet inhibiting tissue ingrowth. Alternatively, the side of the foldable sheet anticipated for exposure to internal body organs may be coated with a well-known antifibrogenic coating for inhibiting tissue ingrowth and the formation of lesions.

Tissue aperture repair device 12 can be used in both open surgery and minimally invasive laparoscopic procedures. When used in open surgery, the stiffener is inserted through small diameter opening 19 into device cavity 18 during the manufacturing process. The device cavity is sealed, and the device is packaged for use by the physician. When the device is used in minimally invasive laparoscopic procedures, device cavity 18 communicates with opening 19 that is centrally positioned and extended by collar 46 in foldable sheet 13 of material for insertion of a percutaneous introducer therethrough. It is also contemplated that opening 19 can be formed in the second sheet of material. The introducer is inserted in opening 19 of the cavity to position the repair device against the tissue aperture.

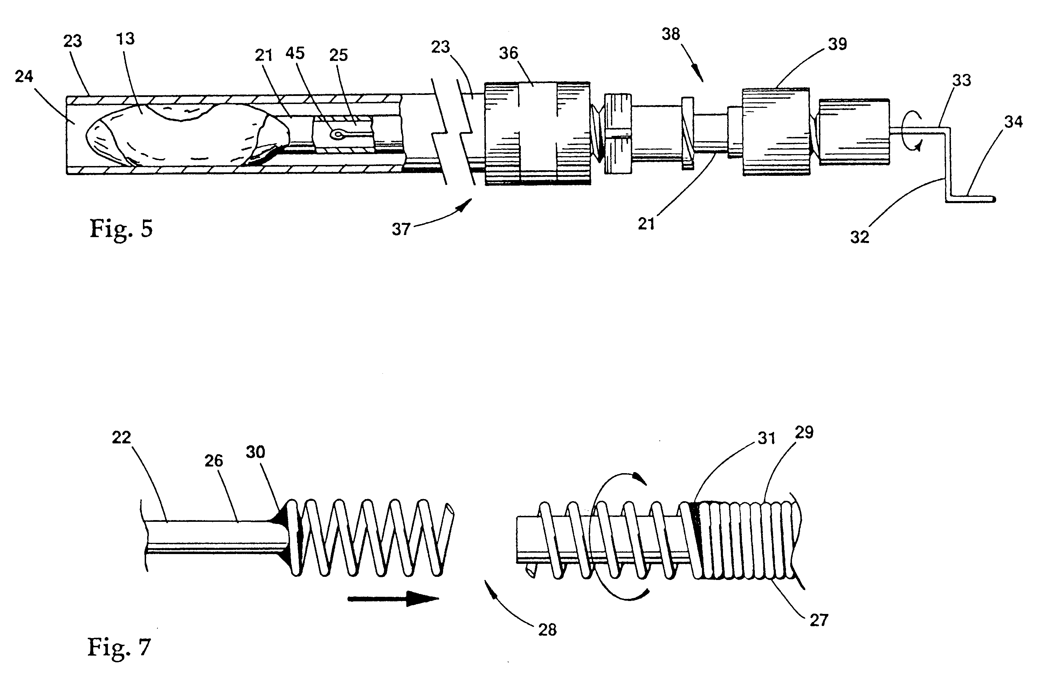

Depicted in FIG. 4 is a partially sectioned side view of tissue aperture repair device 12 with collar 46 and opening 19 centrally positioned in foldable sheet 13 and flared distal end 20 of percutaneous introducer 21 positioned therethrough. As previously described, device cavity 18 is formed between foldable and second sheets 13 and 16 of material bounded by the sheets and circumference 15 of the foldable sheet. Percutaneous introducer 21 includes hollow passageway 25 extending longitudinally therethrough for the insertion and passage of stiffener 22. The foldable and second sheets of material are folded together and wrapped around and about distal end 20 of the introducer and inserted into hollow longitudinal passageway 24 of containment sheath 23 as depicted in FIG. 5. The containment sheath with the tissue aperture repair device contained therein is inserted through a well-known trocar access sheath (not shown) which is introduced into the peritoneal cavity of the patient using a well-known surgical procedure.

Referring again to FIG. 4, when the folded tissue aperture repair device is extended from the distal end of containment sheath 23 into the peritoneal cavity of the patient, elastic stiffener wire 22 is passed through introducer 21 and into device cavity 18. By way of example, the elastic stiffener wire comprises approximately a 110 cm length of Series 302 or 304 stainless steel wire approximately 0.036 cms (.014") in diameter. The length of this wire is approximately five times the circumference (approximately 22 cm) of unfolded sheet 13 of material in unfolded shape 14. The distal end of the stiffener wire is rolled and soldered to form bead 45, which prevents the distal end of the wire from catching or snagging on the sheets of material. This length of elastic stiffener wire is passed through the percutaneous introducer and into device cavity 18, which expands and conforms to circumference 15 of foldable sheet 13 of material as depicted in FIG. 6.

As depicted in FIG. 6, proximal end 26 of elastic stiffener wire is attached to wire guide 27 with detachable wire coil fastener 28. An enlarged view of wire coil fastener 28 attaching proximal end 26 of elastic stiffener wire 22 and distal end 29 of wire guide 27 is depicted in FIG. 7. The wire coil fastener comprises two segments of a 0.097 cms (.038") outside diameter coil of 0.02 cms (.008") stainless steel wire. One segment is attached to proximal end 26 of elastic stiffener wire 22, and the other segment is formed from the turns of wire guide 27. The wire guide segment of the wire coil fastener is formed by separating the coil turns of the wire guide about distal end 29 and fixedly positioning the separated turns using, for example, well-known silver solder bead 31. The segment of the wire coil fastener attached to proximal end 26 of the stiffener is affixed by silver solder bead 30 with the turns of the segment wound in the same direction. The wire guide and stiffener are interconnected by interleaving the turns of the wire coil fastener segments. By way of example, wire guide 27 has a 0.064 cms (.025") outside diameter and is commercially available from several medical device manufacturers such as Cook Incorporated, Bloomington, Indiana. The length of the wire guide is approximately 65 cm with proximal end 32 including two right angle corners 33 and 34 as depicted in FIG. 5 for rotating the wire guide in either a clockwise or counterclockwise direction. To detach elastic stiffener wire 22 from wire guide 27, proximal end 32 of the wire guide is rotated to disengage the interleaved segments of wire coil fastener 28 as depicted by the arrows in FIG. 7. When disengaged, the stiffener wire engages the circumference of device cavity 18.

By way of example, introducer 21 comprises a 50 cm length of polytetrafluoroethylene material tube having an outside diameter of approximately 0.216 cms (.085") with a wall thickness of approximately 0.038 to 0.05 cms (.015-.020"). As depicted in FIG. 4, distal end 20 of the introducer tube is flared in a well-known manner for retainage in device cavity 18. Typically, the distal end of the introducer tube is inserted through collar 46 and opening 19 of the foldable sheet of material and then flared in a well-known manner. Collar 46 is further affixed to the introducer tube with silk suture material 47 wound thereabout, tied, and glued with medical grade adhesive. Collar 46 and suture material 47 transversely position the unfolded and second sheets of material with respect to the introducer. The second sheet of material is then attached to the circumference of the foldable sheet. The foldable and second sheets of material are folded around and about the introducer tube and inserted into containment sheath 23. By way of example, containment sheath 23 comprises a 35 cm length of polytetrafluoroethylene material tube with an outside diameter of 0.3 cms (.120") and a wall thickness of approximately 0.0254 to 0.038 cms (.010-015"). The containment sheath has a well-known gas-tight Luer lock connector 36 attached about proximal end 37 thereof. Similarly, proximal end 38 of introducer tube 21 includes a well-known gas-tight Luer lock connector 39 which engages Luer lock connector 36 of the containment sheath and allows passage of wire guide 32 therethrough.

When the repair device is inserted into the peritoneal cavity of a patient and in its unfolded shape 14, the device is positioned over tissue aperture or hernial rings 1-3 with introducer 21 as depicted in FIG. 2. When properly positioned, the unfolded repair device is attached to the tissue surrounding the hernial rings using, for example, well-known suture material or helical coil fasteners 40. In preparation of positioning the repair device over the hernial ring, the physician places two helical coil fasteners opposite each other and centered over the hernial ring at a distance greater than the diameter of the repair device. These two helical coil fasteners serve as markers for the placement of the device. When the repair device is in a satisfactory position with respect to the marker fasteners, suture material or other helical coil fasteners are inserted into the peritoneal cavity via another trocar access sheath and turned through the repair device and into the tissue surrounding the hernial ring using other well-known minimally invasive surgical instruments. When sufficiently attached to surrounding tissue, the containment sheath is brought up against the distal end of the introducer and foldable sheet of material to pull the distal end of the introducer from collar 46 and opening 19 in foldable sheet of material 13. Wire guide 27 is removed from the containment sheath, and the foldable sheet 13 of material and flared distal end 20 of the introducer is pulled against distal end 35 of sheath 23 as depicted in FIG. 6. When the foldable sheet of material is in the unfolded shape with elastic stiffener wire contained in device cavity 18, flared distal end 20 of the introducer is readily pulled out of collar 46 and opening 19 of the folded sheet 13 by engaging the distal end of sheath 23. Alternatively, the repair device is first removed from the distal end of the introducer by engaging the sheath and pulling the introducer out of the device cavity opening. The detached device is then positioned at least partially over the hernial rings and affixed to the surrounding tissue with other surgical instruments passed through other trocar sheaths.

Another procedure for affixing the unfolded repair device to the tissue surrounding the hernial rings is to include an affixation suture loop 41 to second sheet 13 of material, as depicted in FIG. 6. The repair device includes this suture loop which is passed through containment sheath 23. With one affixation technique, the affixation suture loop is passed through the hernial ring and percutaneously drawn through the abdominal wall and skin of the patient. The repair device is positioned at least partially covering the hernial rings, and the affixation suture is pulled and affixed to the surface of the skin using a well-known percutaneous surgical technique. Depending on the position of the tissue apertures or hernial rings, no additional helical coil fasteners or sutures are required for attaching the repair device to the tissue surrounding the apertures. Alternatively, the folded tissue aperture repair device is percutaneously inserted through the tissue aperture or hernial ring and then extended from the containment sheath. The repair device is extended to its unfolded shape, and the introducer and sheath are pulled back through the insertion site with the affixation suture extending from the folded sheet of material and to the surface skin of the patient for affixation thereto.

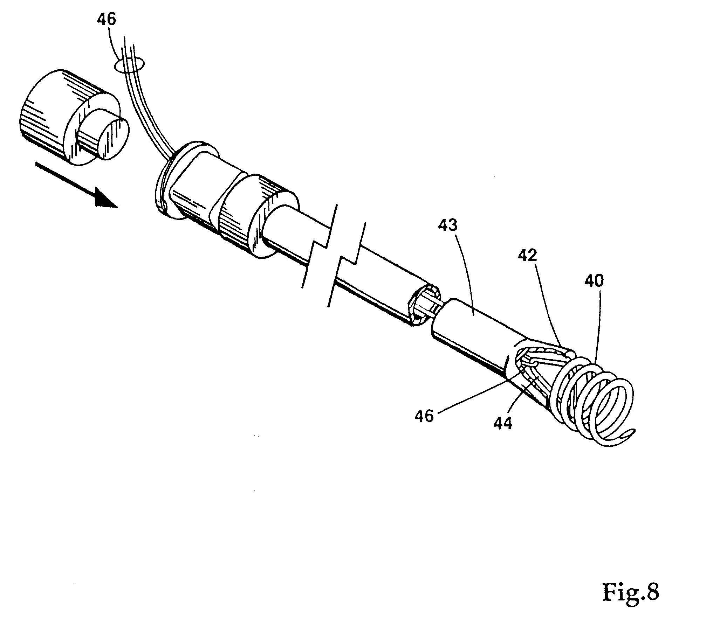

Depicted in FIG. 8 is a helical coil fastener 40 inserted into flared distal end 42 of application cannula 43. By way of example, the helical coil fastener preferably comprises two and a half turns of 0.046 cms (.018") stainless steel wire approximately 0.38 cms (.15") in length with a 0.127 to 0.152 cms (.050-.060") therebetween. The diameter of the helical coil fastener is approximately 0.317 cms (.125"). Eyelet 44 of the fastener extends from the proximal end of the turns and is approximately 0.635 cms (.250") in length. Application cannula 43 comprises a twelve gauge stainless steel cannula having an outside diameter of 0.3 cms (.120") and an inside diameter of approximately 0.24 cms (.095"). Flare 42 about the distal end of application cannula includes a major diameter of approximately 0.38 cms (.150") and a minor diameter of approximately 0.165 cms (.065"). The application cannula is long enough for insertion through a trocar access sheath. The eyelet is secured in the flared distal end of the application cannula using, for example, a loop 46 of suture material. The helical coil fastener is threaded through the foldable and second sheets of material and into the surrounding tissue with rotation of the application cannula. When secured in tissue, the fastener is released by pulling loop 46 of suture material free of the fastener eyelet and drawing the application cannula from the eyelet.

It is to be understood that the above-described tissue aperture repair device is merely an illustrative embodiment of the principles of this invention and that other tissue aperture repair devices may be devised by those skilled in the art without departing from the spirit and scope of this invention. It is contemplated that the tissue aperture repair device is readily usable in both open surgery and minimally invasive surgical procedures. In open surgery, the repair device includes the elastic stiffener wire already positioned in the device cavity or merely affixed to the circumference of the foldable sheet of material. The elastic stiffener material may also comprise a nickel-titanium alloy such as commercially available nitinol, which possesses a superelastic property to readily maintain the device in its unfolded shape. As a result, the number of turns of the stiffener wire around the circumference of the device can be significantly reduced. Furthermore, the elastic stiffener wire may extend across the diameter of the repair device. Several stiffener wires may be crisscrossed to suit any unfolded shape desired. A crisscrossed configuration is ideally suited for a rectangularly shaped tissue aperture repair device. It is further contemplated that the unfolded shape of the tissue aperture repair device can take on any configuration desired by the physician. Percutaneous insertion of the repair device is contemplated using several different techniques as briefly described above. Furthermore, insertion can be from the patient skin surface through a tissue aperture or hernial ring into the peritoneal cavity. The device is unfolded and drawn against the aperture similar to an extended umbrella. The device may be anchored or sutured to the surrounding tissue or simply fixedly positioned using the affixation suture extending from the foldable sheet. Other techniques for percutaneous positioning include well-known intra-cavity approaches. Furthermore, this tissue aperture repair device is not only suited for peritoneal cavity hernias but for any other tissue aperture occurring anywhere in the body such as the thoracic cavity.

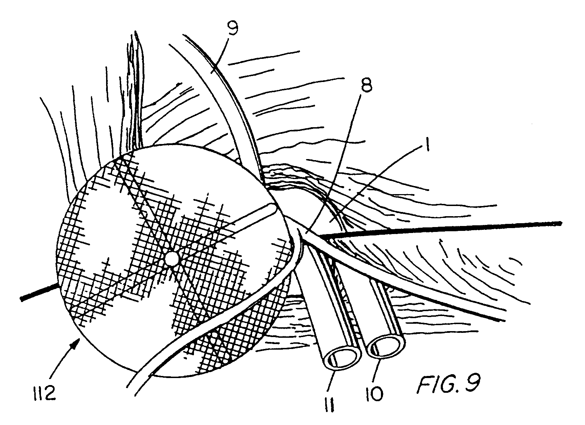

After a hernia the musculature around the inguinal canal is weakened, thereby increasing the risk of recurrent hernia. This may be eliminated by inserting, as shown in FIG.9, a supporting device generally designated 112 across the major part of the hernial ring, thereby preventing the peritoneum from penetrating through one of the hernial rings 1 to 3. The device 112 has such an appropriate size that it may abut on part of the musculature of the abdominal cavity and strengthen it, and that the spermatic cord 8, the femoral artery 10 and vein 11 together with the epigastric vessel 9 may concurrently pass freely by the device.

The barrier material of the device may be a guard of flexible synthetic material, in the illustrated design a net 113 of polypropylene, mersutures or a biodegradable material such as a resorbable polyester, e.g. vicryl or resomer. The barrier material may alternatively be a thin, preferably circular piece of PTFE, e.g. gore-tex (soft tissue patch). Net 113 may be kept spread, mainly in a plane shape by a stiffener 114 consisting of a comparatively thin wire of the alloy Nitinol that is a shape memory alloy which at an increased temperature e.g. at 500°C may be set in a predetermined shape. By an appropriate composition of the alloy the transformation temperature may be selected to be e.g. 10°C so that the wire retains its superelasticity and stiffness during the manipulation and insertion into the abdominal cavity.

Stiffener 114 is advantageously composed of two wires either of which, as the predetermined shape, is formed as an elongate flattened loop 115. Due to this round shape the stiffener has no sharp edges or ends that may grasp and damage the tissue in the abdominal cavity. The net 113 is fixed to stiffener 114 in that the meshes are stuck in over the wire in loop 115 at uniform interspaces, and the two loops 115 extend transversely to each other in order to impart to the net the greatest possible stiffness.

In the middle of the net 113 a fastening means 116 is cast around loops 115 and the net proper. The means 116 may be made from silicone or a biodegradable polymer, such as synthetic or natural polymers (obtainable from e.g. Boehringer Ingelheim), or a resorbable polyester, e.g. vicryl or Resomer. The means 116 is on one side of the barrier material provided with a soft curvature 117 that cannot cause damage to the intestines in the abdominal cavity, and on the other side of the net means 116 has a pin 118 from whose end face a thread 119 extends which may for instance be made from vicryl.

The stiffener is designed as a loop extending along the periphery of the net, which loop may be connected with the fastening means 116 through a number of spokelike arms. The stiffener may be made from stainless steel wire as well, or from a resilient plastics material.

Fig. 12 shows an apparatus for the insertion of the supporting devices 112 and which is composed of an internal carrying tube 120 extending longitudinally through an external guide tube 122 that is somewhat shorter than tube 120. Pin 118 on fastening means 116 and the inner diameter of carrying tube 120 conforms to each other so that the pin upon insertion at one end of the carrying tube will be fixed thereon. The other end of the carrying tube has a radially protruding abutment 121 to confine the displacement of guide tube 122. The thread 119 that is considerably longer than tube 120 is passed centrally therethrough.

With reference to the very simplified Figs. 12 to 16 it will now be described how the device may be operated into the patient.

The abdominal cavity is inflated with gas through a trocar so that the intestines are not positioned in the vicinity of the hernial ring. Then a trocar sheath 123 is percutaneously passed through the abdominal wall that may be composed of musculus obliquus externus and internus 124, musculus transversalis 125 and peritoneum 126. The hernial sac is then reduced.

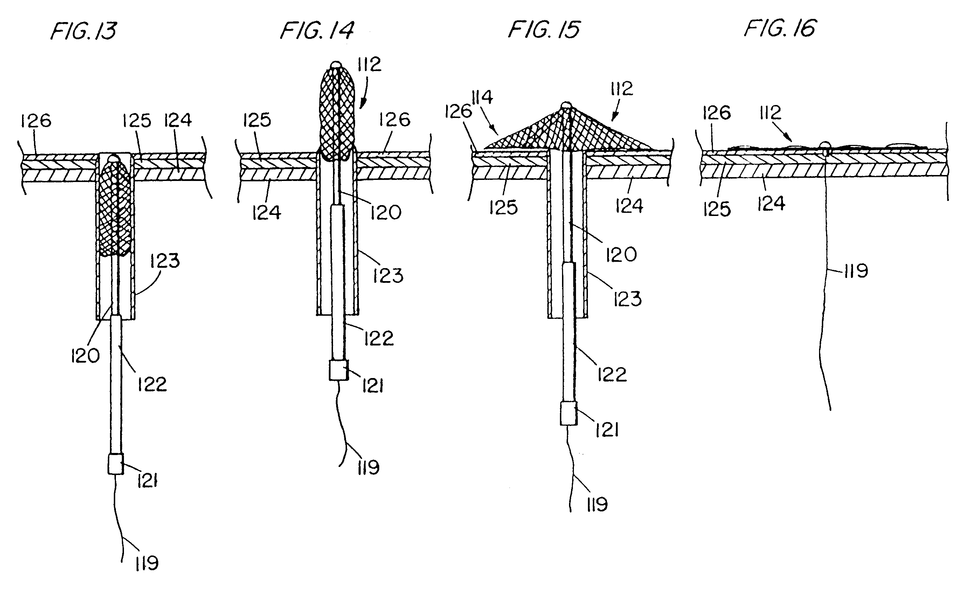

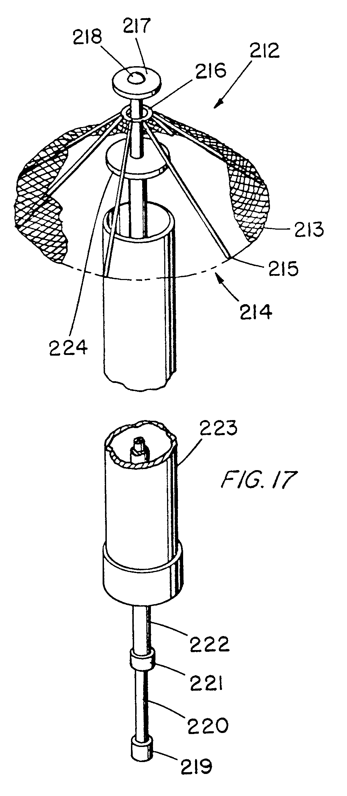

The supporting device mounted at the end of the carrying tube 120 is introduced into the trocar sheath 123 while simultaneously folding the network 113 and stiffener 114 round carrying tube 120, thereby allowing the device to pass through the trocar sheath 123. This folding-up has been made possible by the flexibility of the stiffener or by the circumstance that the stiffener is made from a shape memorizing alloy having superelastic properties. The apparatus is now pushed through sheath 123 and into the abdominal cavity, as illustrated in Fig. 14. When the edge of net 113 is freed from the internal end of sheath 123, stiffener 114 straightens the net as illustrated in Figs. 15 and 16 until the net is substantially plane. This spreading-out may be effected as a consequence of the resiliency or stiffness of the stiffener.

When the supporting device 112 has been introduced into the abdominal cavity the guide tube 122 is moved inwardly to abut on fastening means 116, following which an inwardly directed push on guide tube 122 releases the supporting device 112 from carrying tube 120. Then, tubes 120 and 122 are at first removed and subsequently the trocar sheath 123 so that the tissue and the skin, not shown, close about thread 119 that has been pulled outwardly so that the supporting device 112 abuts on peritoneum 126 and bars hernial rings 1, 2 and 3.

If it is desired that the tissue of the abdominal cavity should grow together with net 113 and stiffener 114, the side of the barrier material facing away from the abdominal cavity may be coated with fibrinogen.

As mentioned above, it is also possible to insert the supporting device 112 through a trocar sheath passed through the abdominal cavity at a distance from the hernial ring. Some manipulation is, however, necessary in this case in order to position the supporting device at the hernial ring.

When the supporting device is to be used to support the abdominal wall in the area where the wall has been weakened by the insertion of the trocar sheath proper, the device may have more diminutive dimensions than in hernia applications. It will normally be sufficient if the barrier material in the spread-out condition is a few times wider than the diameter of the trocar sheath. This smaller device may be inserted and positioned in the same manner as described above with reference to FIGs.13 to 16.

Naturally, the barrier material does not need to have the circular shape shown on the drawings but may be given any appropriate shape, such as oval , crescentic or rectangular.

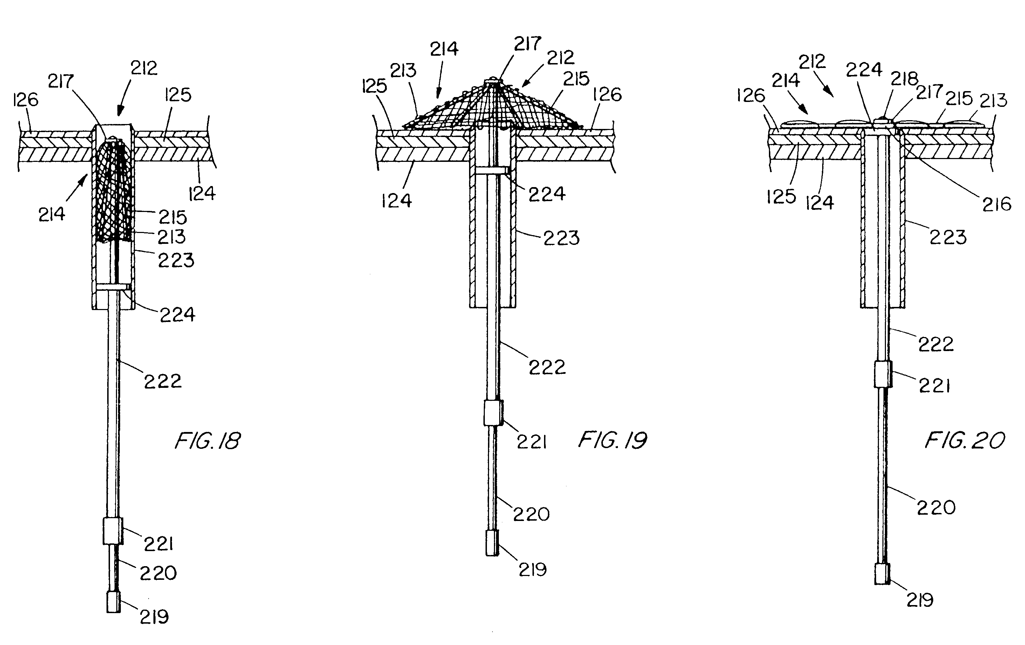

In the embodiment of FIGs.17 to 20, the stiffeners are made of a biodegradable plastmaterial, e.g. polyglycolides/polylactides (PGA/PLA), polyesteranides or polyhydroxybutyrat/polyhydroxyvalerat (PHB/PHV). The fastening means is in this case two rings with different diameters. The stiffeners do not reach the center of the barrier material, but the ends of the stiffeners closest to the center forms a circle with a smaller diameter than the small part of the circumferential fastening means mounted on the same side of the barrier material.

The stiffeners and the fixed part of the fastening means allows the barrier material to be deformed in order to pass through the introducer sheath pushed by the internal carrying tube. When pushed out of the introducer sheath, another part of the fastening means with a diameter close to the diameter of the introducer sheath and bigger than the diameter of the first part of the fastening means is advanced by a coaxially system.

When the other part of the fastening means is pressed against the first part and locked to said first part of the fastening means, the barrier unfolds to the spread-out condition.

## Claims

1. Tissue aperture repair apparatus (12) for providing a support for a weakened area (1,2,3) of a wall of a patient, said apparatus (12) comprising support sheet means (13) which, when in an expanded condition, serves to cover and protect the said weakened area, (1,2,3) and resilient means (22) for urging the support sheet means (13) to the expanded condition, characterised in that the resilient means (22) extends circumferentially about the support sheet means (13) in order to expand the latter to apply tension in the expanded condition.

2. Apparatus according to claim 1, characterised in that when used for open surgery, the resilient means (22) is fixed to the peripheral edge of the sheet means (13).

3. Apparatus according to claim 1, characterised in that the sheet means (13) comprises an attachment cavity (18) extending to the peripheral edge of the sheet means (13), and in that the resilient means (22) is in the form of a spring loaded wire (22) insertable into the cavity (18) in order to expand the sheet means (13).

4. Apparatus according to claim 3, characterised in that when used for minimally invasive surgery, the apparatus further comprises an introducer (21) through which the sheet means (13) is to be introduced in a folded condition into the patient, and through which the wire (22) is to be introduced into the attachment cavity (18).

5. Apparatus according to claim 4, characterised in that the sheet means (13) comprises two sheets (13,16) peripherally joined to form the said cavity (18), and in that means (46,47) are provided to connect the introducer (21) to an aperture (19) in one of the sheets to facilitate entry of the wire (22) into the said cavity (18), and to disconnect the introducer therefrom.

6. Apparatus according to any one preceding claim, further comprising a plurality of mutually angled flexible wires (114) extending centrally from a fastening means (116) on the said one sheet (113).

7. Apparatus according to claim 6, wherein the wire (114) is biodegradable.

8. Apparatus according to claim 6 or 7, characterised in that a thread (119) for fastening the said sheet (113) in a desired position extends from the fastening means (116).

9. Apparatus according to any one of claims 1 to 6 or 8, characterised in that the resilient means (22) is made from a material with superelastic shape memory properties.

## Patentansprüche

1. Vorrichtung (12) zum Wiederherstellen einer Gewebeöffnung zur Unterstützung eines geschwächten Wandbereichs (1, 2, 3) bei einem Patienten, wobei jene Vorrichtung (12) ein Stützplattenmittel (13), das im ausgedehnten Zustand als Abdeckung und Schutz für diesen geschwächten Bereich (1, 2, 3) dient, und ein elastisches Mittel (22) zur zwangsweisen Überführung des Stützplattenmittels (13) in den ausgedehnten Zustand umfaßt, dadurch gekennzeichnet, daß das elastische Mittel (22) sich zur Ausdehnung des Stützplattenmittels (13) um dessen Umfang erstreckt, um es im ausgedehnten Zustand unter Spannung zu setzen.

2. Vorrichtung nach Anspruch 1, dadurch gekennzeichnet, daß das elastische Mittel (22) zum Gebrauch bei offenen Operationen an dem Umfangsrand des Plattenmittels (13) befestigt ist.

3. Vorrichtung nach Anspruch 1, dadurch gekennzeichnet, daß das Plattenmittel (13) einen Befestigungshohlraum (18) umfaßt, der sich bis zum Umfangsrand des Plattenmittels (13) erstreckt, und daß das elastische Mittel (22) als federbelasteter Draht (22) ausgebildet ist, der in den Hohlraum (18) zur Ausdehnung des Plattenmittels (13) einführbar ist.

4. Vorrichtung nach Anspruch 3, dadurch gekennzeichnet, daß die Vorrichtung zum Gebrauch bei minimalinvasiven Operationen weiterhin ein Einführungsinstrument (21) umfaßt, durch welches das Plattenmittel (13) im zusammengefalteten Zustand in den Patienten eingeführt wird und durch welches der Draht (22) in den Befestigungshohlraum (18) eingeführt wird.

5. Vorrichtung nach Anspruch 4, dadurch gekennzeichnet, daß das Plattenmittel (13) zwei zur Ausbildung jenes Hohlraums (18) am Umfang miteinander verbundene Platten (13, 16) umfaßt, und daß Mittel (46, 47) zur Verbindung des Einführungsinstruments (21) mit einer Öffnung (19) in einer der Platten, um die Einführung des Drahts (22) in jenen Hohlraum (18) zu erleichtern, und zur Lösung des Einführungsinstruments von jenem Hohlraum vorgesehen sind.

6. Vorrichtung nach einem der vorhergehenden Ansprüche, weiterhin umfassend eine Vielzahl von gegenseitig abgewinkelten flexiblen Drähten (114), die sich mittig von einem Befestigungsmittel (116) auf jener einen Platte (113) erstrecken.

7. Vorrichtung nach Anspruch 6, worin der Draht (114) biologisch abbaubar ist.

8. Vorrichtung nach Anspruch 6 oder 7, dadurch gekennzeichnet, daß ein Faden (119) zur Befestigung jener Platte (113) in einer gewünschten Lage sich von dem Befestigungsmittel (116) aus erstreckt.

9. Vorrichtung nach einem der Ansprüche 1 bis 6 oder 8, dadurch gekennzeichnet, daß das elastische Mittel (22) aus einem Material mit superelastischen Formgedächtniseigenschaften besteht.

## Revendications

1. Appareil (12) pour la réparation d'une ouverture dans un tissu destiné à fournir un soutien à une zone affaiblie (1, 2, 3) d'une paroi d'un patient, ledit appareil (12) comprenant un moyen à feuilles de soutien (13) qui, lorsqu'il est en état déployé, sert à couvrir et à protéger ladite zone affaiblie (1, 2, 3), et un moyen élastique (22) pour solliciter la moyen à feuilles de soutien (13) vers l'état déployé, caractérisé en ce que le moyen élastique (22) s'étend circonférentiellement tout autour du moyen à feuilles de soutien (13) en vue de déployer ce dernier, afin d'appliquer une tension dans l'état déployé.

2. Appareil suivant la revendication 1, caractérisé en ce que, lorsqu'il est utilisé pour la chirurgie ouverte, le moyen élastique (22) est fixé au bord périphérique du moyen à feuilles (13).

3. Appareil suivant la revendication 1, caractérisé en ce que le moyen à feuilles (13) comprend une cavité d'attache (18) s'étendant jusqu'au bord périphérique du moyen à feuilles (13), et le moyen élastique (22) a la forme d'un fil métallique mis soins contrainte par effet de ressort (22), pouvant être introduit dans la cavité (18) en vue de déployer le moyen à feuilles (13).

4. Appareil suivant la revendication 3, caractérisé en ce que, lorsqu'il est utilisé pour une chirurgie à caractère invasif minimal, l'appareil comprend, en outre, un organe d'introduction (21) à travers lequel le moyen à feuilles (13) doit être introduit dans un état replié dans le patient, et à travers lequel le fil métallique (22) doit être introduit dans la cavité d'attache (18).

5. Appareil suivant la revendication 4, caractérisé en ce que le moyen à feuilles (13) comprend deux feuilles (13, 16) jointes périphériquement pour former ladite cavité (18), et des moyens (46, 47) sont prévus pour relier l'organe d'introduction (21) à une ouverture (19) dans l'une des feuilles afin de faciliter l'entrée du fil (22) dans ladite cavité (18), et pour détacher l'organe d'introduction de l'ouverture (19).

6. Appareil suivant l'une quelconque des revendications précédentes, comprenant, en outre, plusieurs fils métalliques qui se croisent mutuellement (114) et s'étendent au centre à partir d'un moyen de fixation (116) sur ladite feuille (113).

7. Appareil suivant la revendication 6, dans lequel le fil (114) est biodégradable.

8. Appareil suivant la revendication 6 ou 7, caractérisé en ce qu'un fil textile (119) destiné à fixer ladite feuille (113) dans une position souhaitée s'étend à partir du moyen de fixation (116).

9. Appareil suivant l'une quelconque des revendications 1 à 6 ou 8, caractérisé en ce que le moyen élastique (22) est constitué d'une matière ayant des propriétés de mémoire de forme superélastiques.
